# EUROPEAN PATENT APPLICATION

(11) **EP 4 413 975 A1**
(43) Date of publication of application: **14.08.2024**
(21) Application number: 22849903.4
(22) Date of filing: 28.07.2022
(51) Int. Cl.: A61K 9/14, A61K 47/12, A61K 47/22

(54) **AMORPHOUS NANO-MOLECULAR AGGREGATE COMPOSED OF ORGANIC MATERIAL, INORGANIC MATERIAL, OR SALT THEREOF, AND METHOD FOR PREPARING SAME**

(30) Priority: 28.07.2021 KR 20210099485
(71) Applicant: Scai Therapeutics Co., Ltd., Seoul 06524 (KR)
(72) Inventor: KIM, Chul Hwan, Daejeon 35246 (KR); KIM, Kyoung Hee, Daejeon 35246 (KR)
(74) Representative: dompatent von Kreisler Selting Werner - Partnerschaft von Patent- und Rechtsanwälten mbB
(86) International application number: PCT/KR2022/011132
(87) International publication number: WO 2023/008927

(57) **Abstract**

The present invention relates to an amorphous nano-molecular association composed of an organic/inorganic material or a salt thereof, and more specifically, to an amorphous nano-molecular aggregate, which is prepared by applying a shear stress to an organic/inorganic material or a salt thereof, and thus has excellent solubility and permeability to a lipid membrane.

## Description

### [Technical Field]

The present invention relates to an amorphous nano-molecular association composed of an organic/inorganic material or a salt thereof, and more specifically, to an amorphous nano-molecular aggregate, which is prepared by applying a shear stress to an organic/inorganic material or a salt thereof, and thus has excellent solubility and permeability to a lipid membrane.

### [Background Art]

In general, in order to effectively deliver pharmacologically active ingredients, so-called active ingredients, in the form of organic/inorganic materials or salts thereof to a target location in the human body, it is known that the following two conditions must be satisfied.

First, the aqueous solubility of the active ingredient must be secured. Since all fluids in the human body are solutions or dispersions based on water, the aqueous solubility or dispersibility must be sufficiently secured, in order for a drug to be delivered to move in the body.

In addition, the permeability of the active ingredient to the hydrophobic membrane must be secured. Since cells in the human body are surrounded by a hydrophobic membrane, such as a phospholipid membrane, the surface properties of drug molecules or drug structures must be hydrophobic to pass through the membrane, or their size must be very small enough to penetrate a cell membrane.

As a conventional method for permeating these drugs through cell membranes in a molecular state, a method of encapsulating a drug in the form of an emulsion or suspension in microspheres using surfactant/polymer structure as a third material has been widely used.

However, the method of encapsulating this drug has limitations in that the encapsulation process is difficult, the encapsulation yield may be low, and the surfactant or polymer component surrounding the drug must be removed or passed through in order for the drug to be released. In addition, since most of the surfaces of cells or tissues that require permeation are composed of phospholipid components and the like, which are lipophilic, there is a problem in that absorption of the drug is not efficient when the drug is hydrophilic.

In order to solve the problems of the encapsulation method of this drug, many researchers have utilized physicochemical bonding by using a third material as mentioned above, rather than utilizing the structure of the molecule itself.

In order to disperse or dissolve drug molecules in water, a method of making the drug molecules polar to induce polar interactions with water molecules, or a method of covalently or ionicly complexing molecules that are very friendly to water, such as PEG, to a part of the molecular structure, or the like may be utilized.

However, these methods had the inconvenience of changing the molecular structure of the drug, there was a problem of price increase due to the preparation process of these molecules, and also, since the changed molecular structure itself was different from the original molecular structure, the initially intended effect of the drug could be displayed only when it was absorbed in the body and then originally returned to its original molecular state. Thus, there was also a limitation in that the drug cannot maintain the original efficacy because the drug molecules do not maintain a plurality of aggregated states without changing their chemical structure. In addition, it was difficult to penetrate the skin gap of 80 nm in size using a drug structure having a size of several hundred nanometers as in the prior art.

Accordingly, various methods for preparing this drug have been studied. In particular, as a method for preparing a drug in nanometer size, there were a top-down method (top-down process) technology such as high pressure homogenization, milling and a piston-gap homogenizer, and bottom-up method (bottom-up process) technology such as precipitation and self-assembly.

The top-down method (top-down process) is a technology for preparing micrometer-sized particles by reducing the size of particles through a method such as milling, and has disadvantages such as increased cost, risk of contamination, and product damage due to repeated milling. On the other hand, the bottom-up method (bottom-up process) is a technology for growing nanometer-sized structures at the atomic or molecular unit, directly opposite to the top-down method, and is a technology proposed as a way to overcome the limitations of the conventional top-down method of reducing the size from a bulk state to a nanometer size. However, at the current technology level, it is difficult to obtain direct results from the economic advantages of technology at the atomic or molecular unit. In addition, it had the advantages of low cost and simple preparation process through crystal growth, which is a conventional method, but had problems in that only crystalline products could be prepared and a separate compound such as a surfactant had to be added in the excessive growth of crystals and their aggregation.

(Patent Document 1) Korean Laid-open Patent Publication No. 10-2011-0053775 (May 24, 2011), A DISPERSION APPARATUS FOR NANO POWDERS USING INTENSITY FOCUSED ULTRASONICS WAVE AND A DISPERSING METHOD USING THEREOF.

### [Disclosure]

### [Technical Problem]

Accordingly, in order to solve the above-mentioned problems, the present inventors have confirmed a phenomenon in which when drug molecules as pharmacologically active ingredients were dissolved in a solvent and then the molecules were brought very close to each other, polar groups in the molecules behaved as if they were a single entity.

In addition, the present inventors have confirmed that when the polar groups interact with each other due to molecular proximity, the drug molecules are hydrophobic as a whole, but the dispersity may be increased because the surface tension decreases as the size of the structure in which these drug molecules are bonded decreases.

From the above, the present inventors have confirmed that small particles at the molecular level may be prepared in the structure of an amorphous, non-crystalline, nano-sized drug, while being prepared in a bottom-up method rather than a top-down method.

From the above, the present inventors have confirmed that a structure in which molecules with new properties are bonded may be prepared through various methods of reducing an intermolecular distance, such as the preparation of molecular structures using pores in powder or the approach of molecules using flexible rolls, and have completed the present invention.

Therefore, it is an object of the present invention to provide a molecular association of a pharmacologically active ingredient with increased permeability to a phospholipid membrane, by preparing a molecular association with a new structure using polar interactions or hydrogen bonds of molecules without changing the chemical structure of pharmacologically active ingredient molecules to make the surface of the molecular association hydrophobic.

Therefore, the present invention is prepared using a new method rather than the conventional top-down method, and is expected to bring innovation to the field of nanomedicine, ultimately nanotechnology, by improving the problem of shelf-life maintenance period and the problem of too fast dissolution rate due to the particle size of amorphous nanoparticles, despite the advantage of reaching the highest saturation solubility of the conventional amorphous drug nanoparticles. In conclusion, the present invention will become a new innovative technology in the field of 50 nm or less amorphous nanomedicine and nanotechnology.

### [Technical Solution]

In order to achieve the above object, the present invention provides an amorphous nano-molecular association in which an organic material or an inorganic material is physically bonded, wherein when formed as a composition comprising water in the nano-molecular aggregate, the nano-molecular association in the composition has an aggregated structure, and wherein the average particle diameter of the nano-molecular association is 50 nm or less.

In addition, the present invention provides an amorphous nano-molecular association in which a salt of organic material or inorganic material is physically bonded, wherein when formed as a composition comprising water in the nano-molecular aggregate, the nano-molecular association in the composition has an aggregated structure, and wherein the average particle diameter of the nano-molecular association is 50 nm or less.

In addition, the present invention provides a method for preparing the nano-molecular aggregate, comprising steps of: putting a composition comprising an organic material, an inorganic material or a salt thereof, and water into an apparatus capable of applying a shear stress; and then,
applying a shear stress to the composition to prepare a nano-molecular association in which the organic material, inorganic material or salt thereof is physically bonded.

### [Advantageous Effects]

By preparing a molecular association with a new structure using polar interactions or hydrogen bonds without the inconvenience of changing the molecular structure of an organic material, an inorganic material or a salt thereof such as a pharmacologically active ingredient to make the surface of the molecular association hydrophobic, the present invention may have the advantages of not only increasing the permeability to a phospholipid membrane, but also showing the initially intended drug effect as it is because the original molecule and chemical properties themselves are the same, and saving the preparation cost because the preparation method is simple.

Due to these advantages, the molecular association of the present invention may be used in various pharmaceutical compositions.

### [Description of Drawings]

Figure 1 shows a schematic diagram of an apparatus for preparing the molecular association of pharmacologically active ingredients according to an embodiment of the present invention.
Figure 2 shows a schematic diagram of an apparatus for preparing the molecular association of pharmacologically active ingredients according to another embodiment of the present invention.
Figure 3 is a graph showing the results of measuring NOESY, which is a two-dimensional nuclear Overhauser effect (NOE) spectrum, for the pharmacologically active ingredients according to an embodiment of the present invention.
Figure 4 is a graph showing the results of measuring NOESY, which is a two-dimensional NOE spectrum, for the molecular association of pharmacologically active ingredients according to an embodiment of the present invention.
Figure 5 shows a comparison of NMR spectra according to an embodiment of the present invention.
Figure 6 shows a comparison of FT-IR spectra according to an embodiment of the present invention.
Figures 7A to 7C show comparisons of HPLC measurement results according to an embodiment of the present invention.
Figure 8 shows a comparison of NMR spectra according to another embodiment of the present invention.
Figure 9 shows a comparison of FT-IR spectra according to another embodiment of the present invention.
Figure 10 shows a comparison of HPLC measurement results according to another embodiment of the present invention.
Figure 11 shows a comparison of FT-IR spectra according to another embodiment of the present invention.
Figure 12 shows a comparison of FT-IR spectra according to another embodiment of the present invention.
Figure 13 shows a TEM picture of the molecular association according to another embodiment of the present invention.
Figure 14 is a graph showing XRD measurement results of the molecular aggregates according to another embodiment of the present invention.
Figure 15 is a graph showing XRD measurement results of the molecular aggregates according to another embodiment of the present invention.
Figure 16 is a graph showing DSC measurement results of the molecular aggregates according to another embodiment of the present invention.
Figure 17 shows a TEM picture of the molecular association according to another embodiment of the present invention.
Figure 18 shows a TEM picture of the molecular association according to another embodiment of the present invention.
Figure 19 shows a TEM picture of the molecular association according to another embodiment of the present invention.
Figure 20 shows 3D hologram pictures of the phospholipid membrane permeation performance of the molecular aggregates according to another embodiment of the present invention.
Figure 21 shows a schematic diagram of an Ussing chamber for measuring corneal permeation performance of the molecular association according to another embodiment of the present invention.

### [Best Mode]

Herein is provided a molecular association of an organic material, an inorganic material or a salt thereof, which has the structure of an amorphous, non-crystalline, nano-sized drug, while preparing small particles at the molecular level in a bottom-up method rather than a top-down method, and has increased permeability to a phospholipid membrane, by preparing a molecular association with a new structure using polar interactions or hydrogen bonds without changing the chemical structure of the organic material, inorganic material or salt thereof to make the surface of the molecular association hydrophobic.

Hereinafter, the present invention will be described in more detail.

### Apparatus for preparing a molecular association of an organic material, an inorganic material or a salt thereof

The apparatus for preparing a molecular association of an organic material, an inorganic material or a salt thereof as mentioned in this specification is characterized in that a solution containing an organic material, an inorganic material or a salt thereof is put into the apparatus, and then a shear stress is applied to the solution containing the organic material, inorganic material or salt thereof to prepare a molecular association of the organic material, inorganic material or salt thereof.

As a method for preparing a drug in nanometer size, there were a top-down method technology such as high pressure homogenization, milling and a piston-gap homogenizer, and bottom-up method technology such as precipitation and self-assembly.

The preparation technology of the top-down method is a technology for preparing micrometer-sized particles by reducing the size of particles through a method such as milling, and has disadvantages such as increased cost, risk of contamination, and product damage due to repeated milling.

On the other hand, the preparation technology of the bottom-up method such as precipitation had the advantages of low cost and simple preparation process through crystal growth, but had problems in that only crystalline products could be prepared and a separate compound such as a surfactant had to be added in the excessive growth of crystals and their aggregation.

In addition, in order to well deliver pharmacologically active ingredients in the form of an organic material, an inorganic material or a salt thereof to the target in the human body, the aqueous solubility of the organic material, inorganic material or salt thereof and its permeability to a hydrophobic membrane must be secured. As a conventional method for permeating these drugs through cell membranes in a molecular state, a method of making the drug polar to induce polar interactions with water molecules, or a method of covalently or ionicly complexing molecules that are very friendly to water, such as PEG, to a part of the molecular structure, or the like has been widely used.

However, there were limitations in that these methods had the inconvenience of changing the molecular structure of the drug, and also, since the changed molecular structure itself was different from the original molecular structure, the initially intended effect of the drug could be displayed only when it was absorbed in the body and then originally returned to its original molecular state.

In order to solve the above-mentioned problems, the present inventors have confirmed a phenomenon in which when drug molecules in the form of an organic material, an inorganic material or a salt thereof were dissolved in a solvent and then the molecules were brought very close to each other, polar groups in the molecules interacted to form a molecular association and behaved as if they were a single entity.

From the above, the present inventors have confirmed that when the polar groups interact with each other due to molecular proximity, the drug molecules are hydrophobic as a whole, but the dispersity may be increased because the surface tension decreases as the size of the structure in which these drug molecules are bonded decreases.

The present inventors have confirmed that a molecular association in which molecules with new properties are bonded may be prepared through various methods of reducing an intermolecular distance, such as the preparation of molecular structures using pores in powder or the approach of molecules using flexible rolls, and have completed the present invention.

That is, the present invention is to prepare small particles at the molecular level in the structure of an amorphous, non-crystalline, nano-sized drug, while preparing them in a bottom-up method rather than a top-down method.

First, the present invention provides an apparatus, in which a solution containing an organic material, an inorganic material or a salt thereof is put into the apparatus, and then a shear stress is applied to the solution containing the organic material, inorganic material or salt thereof to prepare a molecular association of the organic material, inorganic material or salt thereof. The apparatus for preparing a molecular association of an organic material, an inorganic material or a salt thereof may be used without particular limitation as long as the molecular association of the organic material, inorganic material or salt thereof according to the present invention may be prepared by applying a shear stress to a solution containing the organic material, inorganic material or salt thereof, but preferably those applying a shear stress using a roll mill process or a ball mill process may be used.

When the solution containing the organic material, inorganic material or salt thereof is prepared, it may be prepared in an oil phase. In this case, as a solvent for preparing it in the oil phase, any one or more of oils derived from grain extracts such as castor oil, MCT oil, soybean oil and peanut oil, and oils derived from extracts of medicinal herbs such as ginseng, camellia, green tea and Korean angelica root, showing pharmacological effects, may be used, but are not necessarily limited thereto.

In addition, in the present invention, the organic material, inorganic material or salt thereof may be used as a pharmacologically active ingredient, and may be used without particular limitation as long as it is a pharmaceutically useful material or a material having a medical effect. As an example, cyclosporin A, paclitaxel, docetaxel, decursin, meloxicam, itraconazole, celecoxib, capecitabine, travo, prost, isoflavone, diclofenac sodium, tyrosine kinase inhibitors such as sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, various chokic acids such as deoxycholic acid and ursodeoxycholic acid, or sodium or potassium salts thereof; steroids such as prednisolone substituted with fluorine or present as hydrogen; fragrance oils such as eucalyptus oil, lavender oil, lemon oil, sandalwood oil, rosemary oil, chamomile oil, cinnamon oil and orange oil; alpha-bisabolol, vitamin A (retinol), vitamin E, tocopheryl acetate, vitamin D, vitamin F or derivatives thereof, or the like may be used, or a combination thereof may be used.

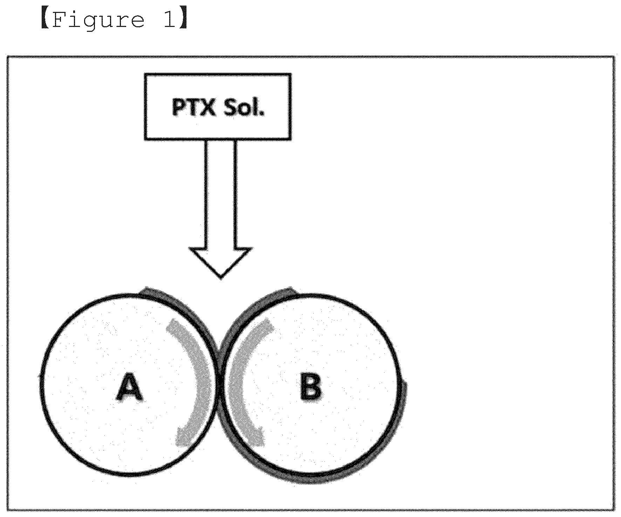

As a specific embodiment of the apparatus for applying a shear stress, a schematic diagram for explaining an apparatus for preparing the molecular association of an organic material, an inorganic material or a salt thereof according to an embodiment of the present invention is shown in Figure 1.

As shown in Figure 1, the apparatus for preparing the molecular association of an an organic material, an inorganic material or a salt thereof according to an embodiment of the present invention may be provided with a plurality of rolls so as to apply a shear stress to the solution containing the organic material, inorganic material or salt thereof. In this case, the plurality of rolls may be two rolls facing each other, and may further include one or several rolls therein.

Specifically, the solution containing the organic material, inorganic material or salt thereof (for example, indicated as PTX Sol. in Figure 1) may be put between two rolls facing each other (indicated as roll A and roll B in Figure 1) in the apparatus. As the two rolls facing each other rotate with respect to the solution containing the organic material, inorganic material or salt thereof put between the two rolls, a shear stress is applied to the organic material, inorganic material or salt thereof contained in the solution containing the organic material, inorganic material or salt thereof, so that the molecular association of the organic material, inorganic material or salt thereof according to the present invention may be prepared.

In the apparatus for preparing the molecular association of an organic material, an inorganic material or a salt thereof according to an embodiment of the present invention, the distance between the two rolls facing each other may be set to 0.5 to 1000 um. The distance between the two rolls facing each other, for example, may be set to 0.5 um or more, 1 um or more, 2 um or more, 3 um or more, 4 um or more, 5 um or more, or 10 um or more, and may be set to 1000 um or less, 900 um or less, 800 um or less, 700 um or less, 600 um or less, 500 um or less, 400 um or less, 300 um or less, 200 um or less, or 100 um or less, and preferably may be set to 10 to 100 um. If the distance between the two rolls facing each other is less than 0.5 um, the discharge amount between the rolls is too small, resulting in a problem of production speed, and if it is more than 1000 um, the shear stress or compressive stress is too small, resulting in a problem that formation of particles is not easy.

In the case of passing the solution between the rolls as described above, excessive force is applied between the molecules, so that the milling of the top-down method does not occur, but the aggregated particle structure between each molecule is created in the bottom-up method.

In addition, the apparatus for preparing the molecular association according to an embodiment of the present invention may rotate the two rolls facing each other at different speeds, in order to more efficiently transfer a shear stress to the organic material, inorganic material or salt thereof contained in the solution. In this case, the rotational speed of any one of the two rolls facing each other may be 50 to 250 rpm, and the rotational speed of the other may be 200 to 500 rpm. Alternatively, the rotational speed of each of the two rolls facing each other may rotate at a ratio of 1:1.5 to 1:5.

In addition, in the apparatus for preparing the molecular association according to an embodiment of the present invention, the rotational direction of the two rolls facing each other may be set to a co-current direction having the same rotational direction, or may be set to a counter-current direction having different rotational directions.

In addition, the apparatus for preparing the molecular association according to an embodiment of the present invention may repeatedly apply a shear stress or compressive stress to the contents discharged once several times.

In addition, the present invention provides an apparatus, in which a solution containing an organic material, an inorganic material or a salt thereof is put into the apparatus in a first side direction, and another solution is put in a second side direction facing the first side direction, and then a shear stress is applied to prepare a molecular aggregate. The apparatus for preparing the molecular association of the present invention may also be used without particular limitation as long as the molecular association of the present invention may be prepared by applying a shear stress to the solution containing the organic material, inorganic material or salt thereof, but preferably those applying a shear stress using a roll mill process or a ball mill process may be used.

In the present invention, the pharmacologically active ingredients may be the same as mentioned above.

In the present invention, as the water-soluble compounds, any one or more selected from the group consisting of citric acid, carbonic acid, lactic acid, acetic acid, phosphoric acid, ascorbic acid, malic acid, tartaric acid, glutaric acid, succinic acid, maleic acid, fumaric acid, malonic acid, HCl, H₂SO₄, NaH₂PO₄, NaHCO₃, KHCO₃, Na₂CO₃, K₂CO₃, Na₃PO₄, K₃PO₄, NaH₂PO₄, NH₄OH, sodium acetate (NaOAc), KOH, NaOH and Ca(OH)₂ may be used.

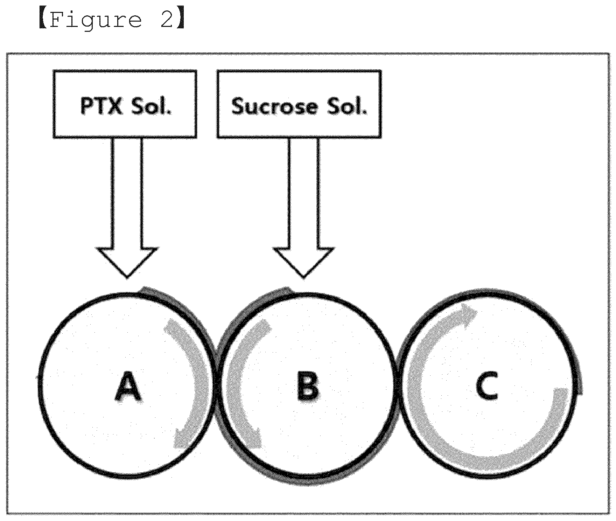

As a specific embodiment of the apparatus for applying a shear stress, a schematic diagram for explaining an apparatus for preparing the molecular association according to an embodiment of the present invention is shown in Figure 2.

As shown in Figure 2, the apparatus for preparing the molecular association according to an embodiment of the present invention may be provided with a plurality of rolls so as to apply a shear stress to a solution containing an organic material, an inorganic material or a salt thereof. In this case, the plurality of rolls may be two rolls facing each other (roll A, roll B), and may further include one or several rolls (roll C) as shown in Figure 2.

Specifically, the solution containing the organic material, inorganic material or salt thereof (for example, indicated as PTX Sol. in Figure 2) may be put into the first roll side, and the solution containing the water-soluble compounds (for example, indicated as Sucrose Sol. in Figure 2) may be put into the side of the second roll facing the first roll.

As the two rolls facing each other rotate with respect to the solution containing the organic material, inorganic material or salt thereof and the solution containing the water-soluble compounds, respectively put in different directions of the two rolls, a shear stress is applied to the organic material, inorganic material or salt thereof and the water-soluble compounds contained in these solutions, so that the molecular association of the organic material, inorganic material or salt thereof according to the present invention may be prepared.

In the apparatus for preparing the molecular association according to an embodiment of the present invention, the distance between the two rolls facing each other may be set to 0.5 to 1000 um. The distance between the two rolls facing each other, for example, may be set to 0.5 um or more, 1 um or more, 2 um or more, 3 um or more, 4 um or more, or 5 um or more, and may be set to 1000 um or less, 900 um or less, 800 um or less, 700 um or less, 600 um or less, or 500 um or less, and preferably may be set to 5 to 500 um.

If the distance between the two rolls facing each other is less than 0.5 um, the discharge amount between the rolls is too small, resulting in a problem of production speed, and if it is more than 1000 um, the shear stress or compressive stress is too small, resulting in a problem that formation of particles is not easy.

In addition, the two rolls facing each other may rotate at different speeds, in order to more efficiently transfer a shear stress to the organic material, inorganic material or salt thereof contained in the solution. In this case, the rotational speed of any one of the two rolls facing each other may be 50 to 150 rpm, and the rotational speed of the other may be 200 to 500 rpm. Alternatively, the rotational speed of each of the two rolls facing each other may rotate at a ratio of 1:1.5 to 1:5.

In addition, in the apparatus for preparing the molecular association according to an embodiment of the present invention, the rotational direction of the two rolls facing each other may be set to a co-current direction having the same rotational direction, or may be set to a counter-current direction having different rotational directions.

In addition, the apparatus for preparing the molecular association according to an embodiment of the present invention may repeatedly apply a shear stress or compressive stress to the contents discharged once several times.

In the present invention, the apparatus for preparing the molecular association may further be provided with a third roll for re-applying a shear stress to the solutions passing between the first roll and the second roll. A shear stress may be applied between the second roll and the third roll, and the distance between the second roll and the third roll facing each other may be set to 0.5 to 1000 um. The distance between the two rolls facing each other, for example, may be set to 0.5 um or more, 1 um or more, 2 um or more, 3 um or more, 4 um or more, or 5 um or more, and may be set to 1000 um or less, 900 um or less, 800 um or less, 700 um or less, 600 um or less, or 500 um or less, and preferably may be set to 5 to 500 um. If the distance between the second roll and the third roll is less than 0.5 um, the discharge amount between the rolls is too small, resulting in a problem of production speed, and if it is more than 1000 um, the shear stress or compressive stress is too small, resulting in a problem that formation of particles is not easy.

In addition, the second roll and the third roll may rotate at different speeds, in order to more efficiently transfer a shear stress. In this case, the rotational speed of any one of the second roll and the third roll may be 200 to 500 rpm, and the rotational speed of the other may be 600 to 1200 rpm. Alternatively, the rotational speed of each of the second roll and the third roll may rotate at a ratio of 1:1.5 to 1:5.

In the present invention, the device for preparing the molecular association is not limited to the above method, and it is natural that any device capable of applying a shear stress to an organic material, an inorganic material or a salt thereof may be freely deformed.

### Molecular association of an organic material, an inorganic material or a salt thereof

The present inventors have confirmed a phenomenon in which when drug molecules in the form of an organic material, an inorganic material or a salt thereof were dissolved in a solvent and then the molecules were brought very close to each other, polar groups in the molecules interacted to form a molecular association and behaved as if they were a single entity.

From the above, the present inventors have confirmed that when the polar groups interact with each other due to molecular proximity, the drug molecules are hydrophobic as a whole, but the dispersity may be increased because the surface tension decreases as the size of the molecular association in which these drug molecules are bonded decreases.

The present inventors have confirmed that a molecular association in which molecules with new properties are bonded may be prepared through various methods of reducing an intermolecular distance, such as the preparation of a molecular association using pores in powder or the approach of molecules using flexible rolls, and have completed the present invention.

Accordingly, the nano-molecular association prepared by the apparatus as mentioned in this specification has the following characteristics:
First, the nano-molecular association according to the present invention is characterized in that it is amorphous while having a nanoparticle size, by applying a shear stress to a solution containing an organic material, an inorganic material or a salt thereof, which is a precursor of the molecular aggregate, to reduce an intermolecular distance of the organic material, inorganic material or salt thereof very close, thereby preparing the molecular aggregate. That is, a shear stress is applied between molecules with the same structure to form a nano-sized amorphous molecular association in which they are physically bonded to each other.

In addition, the measured values by chromatography of the molecular association and the organic material, inorganic material or salt thereof, which is a precursor of the molecular aggregate, are the same, and the measured values by spectroscopy of the molecular association and the pharmacologically active ingredient, which is a precursor of the molecular aggregate, have different characteristics.

Common methods for determining the structure of a material include methods through various instrumental analyses, such as elemental analysis, FT-IR, NMR, UV spectroscopy, X-ray, and differential scanning calorimeter (DSC). Among them, NMR and FT-IR are the analytical methods that best show the chemical structure, that is, the atoms constituting the molecule and their connections. Usually, X-ray or DSC is a method widely used to investigate secondary structures such as crystal structures formed by molecules, such as crystal structures. In addition, the microstructure is also analyzed using a scanning electron microscope or a transmission electron microscope.

Among the methods mentioned, NMR may inform the electronic environment of an atomic nucleus, which is determined by the electronic structure of a molecule. The result of the present invention relates to a method for preparing a certain compound and its physical structure, and NMR provides very useful information as a method for determine whether there is no chemical change in the process of forming a physical structure. In the absence of formation or disappearance of chemical bonds, a compound and its physical structure basically have similar NMR spectra. Of course, in order to determine how close the compounds are to each other to form a molecular aggregate, an intermolecular distance of compounds usually located at a distance of 0.5 nm was roughly measured through the nuclear Overhauser effect (NOE) or the two-dimensional NOESY spectrum.

In addition, according to an example of the nano-molecular association according to the present invention, an intermolecular distance of the nano-molecular association may be 10 Å or less. The intermolecular distance refers to a value obtained by measuring the average distance between these molecules based on the molecules constituting a molecular aggregate, and may be measured using, for example, NOE of NMR. When a single hydrogen is excited using a pulse, the phenomenon in which the intensity of hydrogen at a close distance increases is called nuclear Overhauser effect (NOE), which is inversely proportional to the six squares of the distance between hydrogen nuclei. Thus, if intramolecular hydrogens are at a distance, they do not contribute to the increase in peak intensity due to NOE. However, when the molecules come very close together and the distance between intermolecular hydrogens is closer than the distance between intramolecular hydrogens, NOE is observed, indicating that an intermolecular distance becomes very close. It is generally known that NOE is observed when the distance is closer than 1 nm.

In the present invention, it has also been confirmed that the sodium deoxycholate (NaDC) compound and the molecular association obtained in the present invention are gathered on the scale of angstrom (10⁻¹⁰ m) due to the very close intermolecular distance through two-dimensional NOESY NMR.

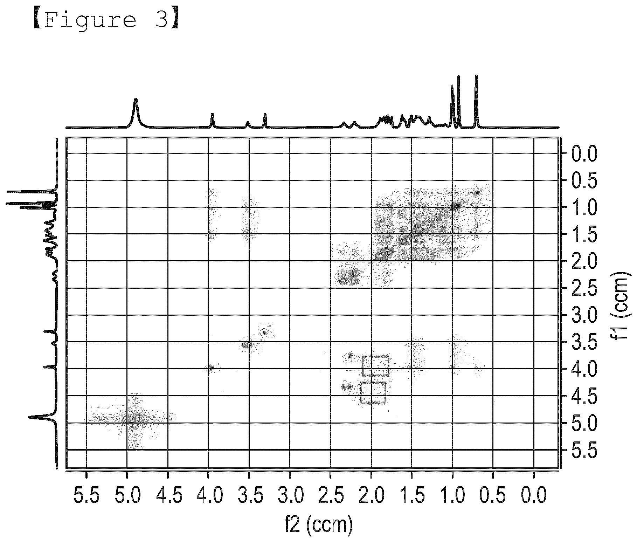

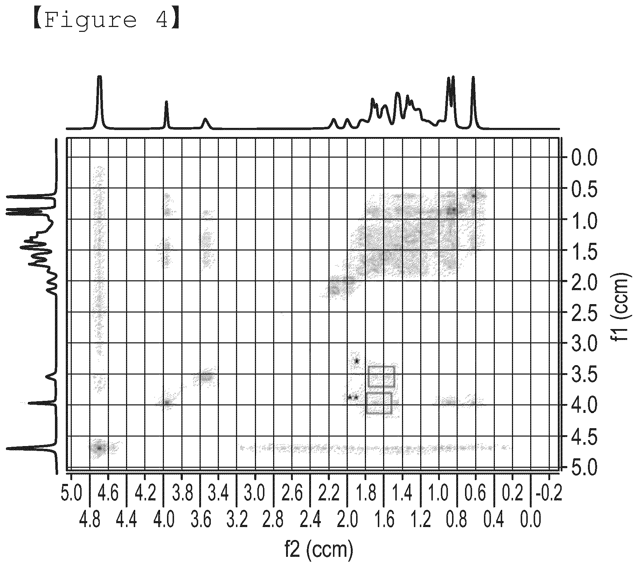

In Figures 3 and 4, the NOESY, which is a two-dimensional NOE spectrum, for NaDC molecules and a molecular association thereof, are shown, respectively. In Figures 3 and 4, parts marked with * and ** indicate the same positions. It can be seen that off-diagonal peaks do not appear in the regions marked with * and ** in the NaDC molecule itself in Figure 3, whereas off-diagonal peaks appear in the regions marked with * and ** in the molecular association of NaDC in Figure 4. This indicates that the distance between two nuclei was far from each other in pure NaDC, but the distance between the two nuclei became closer as the molecular association was formed. From the above, it can be seen that when the molecular association is formed, the intermolecular distance becomes very close. Since it is generally known that the distance required for two hydrogens to exhibit NOE is physically about 6 Å (Angstrom), it can be seen that an intermolecular distance to form the molecular association of the present invention is also within the range of about 6 Å (Angstrom).

In summary, the reason why peaks that did not appear due to the long distance in the molecule itself appear in the nano-molecular association provided by the present invention is caused by the change in the physical position of molecules of an organic material, an inorganic material or a salt thereof bonded to the nano-molecular aggregate. That is, this indicates that an intermolecular distance in the molecular association is very close.

The peak position in the NMR spectrum refers to a frequency at which rotational motion is performed depending on the intensity of a magnetic field applied to an atomic nucleus in a molecule composed of atomic bonds as described above. The intensity of the magnetic field applied to the atomic nucleus varies depending on the property of the surrounding functional groups to push and pull electrons, and as a result, when the intensity of the magnetic field applied to the nucleus increases, it shifts to a higher frequency, and conversely, when the intensity of the magnetic field felt by the nucleus becomes weaker due to the large number of electrons around the nucleus, it reduces in the direction where the frequency of rotational motion of the nucleus is lower. When an association of molecules is physically formed, the density of the electron cloud of the atomic nucleus varies depending on the distance between the molecules with strong interactions. In another method, in a nucleus near a phenyl ring (C₆H₅-) where a ring current is formed under a magnetic field, the intensity of the magnetic field is locally changed by the ring current, and thus the number of revolutions of the nucleus is changed. In addition, even when the electron density is high due to the double bond, such as C=O, the position of the peak may shift up field or down field because the intensity of the magnetic field varies depending on the relative position of the surrounding nucleus and C=O.

In the case of the molecular association prepared according to the present invention, it is physically bonded by the interaction between molecules even without separate binders or additives, and may be substantially composed of an organic material or an inorganic material.

Judging from these points, when comparing the result values measured by NMR of the molecular association prepared according to the present invention and the organic material or inorganic material, which is a precursor of the nano-molecular aggregate, it can be seen that the peak position of the NMR spectrum is partially changed, and this is because there is no change in chemical structure between the nano-molecular association and the organic material or inorganic material, which is a precursor of the nano-molecular aggregate, but a change in physical structure occurs. Specifically, when comparing the result values measured by NMR of the nano-molecular association and the organic material or inorganic material, which is a precursor of the nano-molecular aggregate, it may be determined that they are different when the peak shift is 0.005 ppm or more based on 1H NMR.

On the other hand, in the case of FT-IR, it is known that since the density of electrons constituting the bonds in the molecule is localized, a change in peak appears well when a change in dipole moment occurs during molecular motion, and it is known that peaks in the spectrum are excited by various molecular motions such as stretching and bending. When the polarity of the molecular bond is formed by heteroatom, a lot of peak changes appear. For example, an ether bond (C-O), which is a primary bond between carbon and oxygen, or a carbonyl bond (C=O), which is a secondary bond, shows a stretching band by an infrared laser. In addition, even in the case of carbon and hydrogen, a stretching band appears because it is a bond between different molecules. In the case of stretching, it may be explained by a model in which two masses are connected by a spring, and when the energy to excite the movement of the spring is supplied by infrared rays, peaks appear in the spectrum.

Even when several carbon atoms are connected to each other, various bending peaks such as in-plane vibration and out-of-plane vibration appear when six atoms are bonded, such as in benzene. In particular, in the case of bending motion, various types of molecular motions may be observed by FT-IR spectrum. For example, rocking that moves left and right, scissoring that moves like scissors, wagging that swings back and forth on a plane, twisting/torsion that repeats twisting, and the like occur.

Structures of strongly polar molecules may undergo interactions such as hydrogen bonding, polar interactions or pi-pi stacking, and these interactions cause changes in the position and intensity of peaks in the FT-IR spectrum. Thus, even in the same molecular bonding, the position and intensity of peaks may change depending on the surrounding environment. That is, it may be seen that they are the same molecule when the FT-IR spectrum is the same. In particular, 400 cm⁻¹ to 700 cm⁻¹ is called a fingerprint zone, and if peaks are the same in this zone, they are regarded as the same compound.

It is an object of the present invention to form compounds into a structure of a molecular aggregate, which is a physical aggregate. When the compounds are formed into a molecular aggregate, which is a physical aggregate, in this way, an intermolecular distance in the association is very close, within about 10 Å or 5 Å. It has been found that compound bonds in molecular aggregates undergo molecular motions such as stretching or bending when intramolecular bonds are excited by infrared rays, and in this case, due to another compound that exists within a very close distance from a compound, cases involving various changes, such as the appearance or disappearance of peaks that did not appear in the original compound spectrum, a change in the position of a peak or a decrease or increase in intensity of a peak, appear in the molecular association of the present invention.

That is, when one or more peaks appear or disappear based on the result values measured by the FT-IR, it may be determined that they are different.

When comparing the result values measured by FT-IR of the nano-molecular association prepared according to the present invention and the organic material or inorganic material, which is a precursor of the nano-molecular aggregate, it can be seen that one or more peaks of the FT-IR spectrum appear or disappear, and this is because there is no change in chemical structure between the nano-molecular association and the organic material or inorganic material, which is a precursor of the nano-molecular aggregate, but a change in physical structure occurs. In addition, when comparing the result values measured by FT-IR of the nano-molecular association and the organic material or inorganic material, which is a precursor of the nano-molecular aggregate, it may be determined that they are different when one or more peak positions differ by 5 cm⁻¹ or more. That is, the conversion of compounds into a molecular association is due to a physical action, and this does not mean that a new chemical bond is formed or a chemical bond disappears. Therefore, this change of the spectrum may well explain the fact that a molecular association with a new structure, which was not previously available, was created in the present invention.

However, the chemical structure of the nano-molecular association prepared according to the present invention and the organic material or inorganic material, which is a precursor of the nano-molecular aggregate, is the same as described above. This can be seen from the fact that chromatographic analysis was performed on the nano-molecular association and the organic material or inorganic material, which is a precursor of the nano-molecular aggregate, and the measured values were the same. The measured values by chromatography may be the result values measured by high-performance liquid chromatography (HPLC), and it is observed that the organic material or inorganic material and the molecular association thereof, which is the result provided by the present invention, show peaks at almost the same position and have a retention time within about 10 % (i.e., ±5%). In general, when the column and the mobile phase and the stationary phase are the same, peaks appearing at a time within 10% may be determined as the same material.

Like this, since the nano-molecular association and the organic material or inorganic material, which is a precursor of the nano-molecular aggregate, have different physical structures, most of the peaks of the two target materials are similar or some may be changed in the NMR spectrum, and various types of changes such as appearance and disappearance of peaks and changes in peak intensity may appear in FT-IR. In addition, since the nano-molecular association and the organic material or inorganic material, which is a precursor of the nano-molecular aggregate, have the same chemical structure, the measured values by chromatography may be the same.

In the present invention, NMR measurement of the nano-molecular association and the organic material or inorganic material, which is a precursor of the nano-molecular aggregate, is not particularly limited, but may be performed using, for example, a Bruker 400 MHz Avance.

In the present invention, FT-IR measurement of the nano-molecular association and the organic material or inorganic material, which is a precursor of the nano-molecular aggregate, is not particularly limited, but may be performed using, for example, a Bruker Alpha 2 ATR.

Next, another molecular association derived from drug molecules according to the present invention is characterized in that a shear stress is applied to a solution containing a salt of organic material or inorganic material as a precursor of the nano-molecular association to prepare a nano-molecular association with a structure in which the salt of organic material or inorganic material is physically bonded.

The molecular association prepared in this way is a molecular association in which a pharmacologically active ingredient and a water-soluble compound are bonded, and may be characterized in that the measured values by chromatography of a salt of organic material or inorganic material, which is a precursor of the nano-molecular aggregate, and a molecular association in which the salt of organic material or inorganic material is bonded are the same, and the measured values by spectroscopy of the salt of organic material or inorganic material, which is a precursor of the nano-molecular aggregate, and the molecular association in which the salt of organic material or inorganic material is bonded are different.

In the nano-molecular aggregate, the measured value by chromatography and the measured value by spectroscopy are the same as mentioned above.

Likewise, it is physically bonded by the interaction between molecules even without separate binders or additives, and may be substantially composed of a salt of organic material or inorganic material.

In the present invention, the salt of organic material or inorganic material may be used as a pharmacologically active ingredient, and the salt of the above-mentioned organic material or inorganic material may be used.

As described above, since the salt of organic material or inorganic material, which is a precursor of the nano-molecular aggregate, and the molecular association in which the salt of organic material or inorganic material is physically bonded have different physical structures, most of the peaks of the two target materials are similar and some may be changed in the NMR spectrum, and various types of changes such as appearance and disappearance of peaks and changes in peak intensity may appear in FT-IR.

For example, when comparing the result values measured by NMR of the salt of organic material or inorganic material, which is a precursor of the nano-molecular aggregate, and the molecular association in which the salt of organic material or inorganic material is bonded, it may be determined that they are different when the peak position changes, and specifically, when comparing the result values measured by NMR of the salt of organic material or inorganic material, which is a precursor of the nano-molecular aggregate, and the molecular association in which the salt of organic material or inorganic material is bonded, it may be determined that they are different when the peak shift is 0.005 ppm or more based on 1H NMR.

In addition, when comparing the result values measured by FT-IR of the salt of organic material or inorganic material, which is a precursor of the nano-molecular aggregate, and the molecular association in which the salt of organic material or inorganic material is bonded, they may be determined to be different when one or more peaks appear or disappear, and specifically, when comparing the result values measured by FT-IR of the salt of organic material or inorganic material, which is a precursor of the nano-molecular aggregate, and the molecular association in which the salt of organic material or inorganic material is bonded, they may be determined to be different when one or more peaks differ by 5 cm⁻¹ or more.

In addition, since the salt of organic material or inorganic material, which is a precursor of the nano-molecular aggregate, and the molecular association in which the salt of organic material or inorganic material is bonded have the same chemical structure, the measured values by chromatography may be the same. Specifically, they may be determined to be the same, when the result values measured by HPLC of the salt of organic material or inorganic material, which is a precursor of the nano-molecular aggregate, and the molecular association in which the salt of organic material or inorganic material is bonded have a retention time within 10%.

In addition, since the particle size of the nano-molecular association according to the present invention is very small, the average particle diameter may be 50 nm or less, preferably may be 30 nm or less, more preferably may be 20 nm or less, very preferably may be 15 nm or less, and most preferably may be 10 nm or less, or 5 nm or less. The average particle diameter may be measured through a diffraction experiment, and preferably may be measured using small angle neutron scattering (SANS). Also, images may be measured using transmission electron microscopy. When the average particle diameter of the nano-molecular association exceeds 50 nm, there are problems that dispersibility is poor and transparency and permeability are poor. In addition, the lower limit of the average particle diameter of the nano-molecular association is not particularly limited, but may be about 1 nm or more.

Hereinafter, the preparation method of the present invention will be described in more detail through examples of the present invention. It will go without saying that the present invention is not limited to these examples.

### EXAMPLES

### Apparatus used

### 3 roll mill

| Model | | TX3051 Series |
|---|---|---|
| Motor | | 120V (3P/220V) |
| RPM | | Max 360 |
| Dimension (mm) | | 310(V) × 250(0) × 360(H) |
| Roller | Rotation Rate | 9(F) : 3(M) : 1( 'R) |
| | Temp. Rance | Non (Room Temp) |
| | Gap | 0 ∼ 300 mm |
| | Gap Control Method | Dial Rotation Manual Type (15µ m/l Rotation Fine Control) |
| | Display | Digital (µm) LCD Display (P-M, M-R) |
| Calibration | | Independent Calibration, Front/Back, Left/Right |

### Preparation

### [Example 1]

19.8 mg of NaDC (Sodium deoxycholate; Xi'an Lyphar Biotech Co., Ltd., China) was dissolved in 10 mL of water to prepare an aqueous NaDC solution with a concentration of about 0.2%. As shown in Figure 1, a roll mill composed of two rolls, roll A and roll B, was prepared, and then the prepared NaDC solution was put between rolls A and B at an input speed of 50 ml/min. The rotational speed of the roll A was adjusted to 100 rpm and the rotational speed of the roll B was adjusted to 300 rpm, and the distance between the rolls A and B was set to 10 um.

The obtained aqueous solution was frozen at -50°C, and then freeze-dried at the temperature of -70°C under a pressure of 0.1 bar for 48 hours to remove water. The obtained sample was a white powder.

### [Comparative Example 1]

NaDC powder was obtained through the same process as in Example 1, except that an aqueous NaDC solution was prepared and the roll process for preparing a molecular association thereof was not performed.

### [Example 2]

500 mg of sunitinib-malate (SUNITINIB MALATE, TEVA) was dissolved in 100 mL of ethanol to prepare a sunitinib solution with a concentration of about 0.5%. As shown in Figure 1, it was put into a roll apparatus to prepare a molecular aggregate, wherein the input speed of each solution was 50 ml/min. The rotational speed of the roll A was adjusted to 100 rpm and the rotational speed of the roll B was adjusted to 300 rpm, and the distance between the rolls A and B was set to 10 um.

Thereafter, the aqueous solution recovered through the roll mill was frozen at -50°C, and then freeze-dried at the temperature of -70°C under a pressure of 0.1 bar for 48 hours to remove water, and as a result, a "molecular association of sunitinib/maleate" was obtained in powder form.

### [Comparative Example 2]

Sunitinib maleate powder was obtained through the same process as in Example 2, except that sunitinib maleate was prepared as an aqueous solution and the roll process for preparing a molecular association thereof was not performed.

### [Example 3]

A sample was prepared in the same manner as in Example 2, except that the distance between the rolls A and B was set to 90 um.

### [Example 4]

A sample was prepared in the same manner as in Example 2, except that niclosamide was used instead of sunitinib-malate (SUNITINIB MALATE, TEVA) and the distance between the rolls A and B was set to 90 um.

### [Comparative Example 3]

A sample was prepared in the same manner as in Example 2, except that the distance between the rolls A and B was set to 110 um.

### [Comparative Example 4]

A sample was prepared in the same manner as in Example 4, except that the distance between the rolls A and B was set to 110 um.

### [Example 5]

19.8 mg of DCF-DA (2',7'-dichlorofluorescin diacetate; Sigma-Aldrich Co. Ltd.) was dissolved in 10 mL of water to prepare an aqueous NaDC solution with a concentration of about 0.2%. As shown in Figure 1, a roll mill composed of two rolls, roll A and roll B, was prepared, and then the prepared DCF-DA solution was put between rolls A and B at an input speed of 50 ml/min. The rotational speed of the roll A was adjusted to 100 rpm and the rotational speed of the roll B was adjusted to 300 rpm, and the distance between the rolls A and B was set to 10 um.

The obtained aqueous solution was frozen at -50°C, and then freeze-dried at the temperature of -70°C under a pressure of 0.1 bar for 48 hours to remove water. The obtained sample was a yellow fluorescent powder.

### [Comparative Example 5]

DCF-DA powder was obtained through the same process as in Example 5, except that DCF-DA was prepared as an aqueous solution and the roll process for preparing a molecular association thereof was not performed.

### [Example 6]

A molecular association was prepared in the same manner as in Example 1, except that cyclosporin A was used instead of NaDC.

### [Comparative Example 6]

An API was obtained by the same process as in Example 6, except that cyclosporin A was prepared as an aqueous solution and the roll process of preparing a molecular association thereof was not performed.

### Experimental Example 1: Comparison of chemical/physical structures in Example 1 and Comparative Example 1

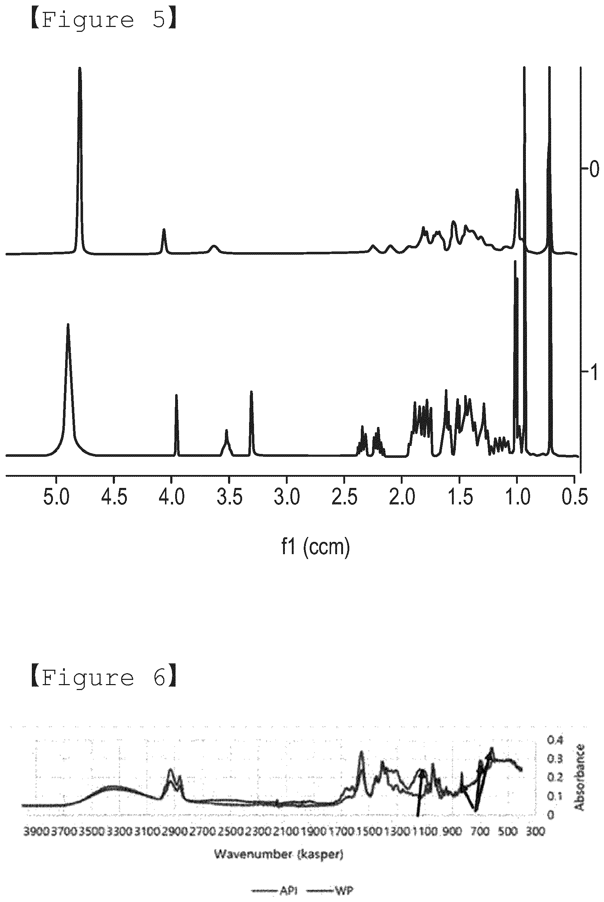

For the NaDC molecular association in powder form prepared in Example 1 and the NaDC itself in Comparative Example 1, the spectra were measured using nuclear magnetic resonance (NMR; Bruker 400 MHz Avance) and Fourier transform-infrared spectroscopy (FT-IR; Bruker Alpha 2 ATR), and the spectrum results according to NMR are shown in Figure 5 and the spectrum results according to FT-IR are shown in Figure 6.

As shown in Figure 5, two spectra of NaDC may be seen. The top spectrum of Figure 5 shows the H-NMR spectrum of NaDC (pure compound, Comparative Example 1), a pharmacologically active ingredient, which is a precursor of the molecular association of the present invention, and the bottom spectrum of Figure 5 shows the H-NMR spectrum of the molecular association of NaDC prepared by the preparation method of the present invention. When comparing the top and bottom spectra of Figure 5, the top spectrum of Figure 5 has very sharp peaks and good resolution, whereas the bottom spectrum of Figure 5 has blunt peaks and an overlapping phenomenon. This difference is because as the structure of the molecular association prepared by the preparation method of the present invention is formed, the molecular weight at which actual molecular motion occurs changes and the spin-spin relaxation time (T2) becomes shorter.

Figure 6 shows the FT-IR spectra of the two materials. When comparing the spectrum of pure NaDC (Comparative Example 1), as indicated in blue, with the spectrum of the molecular association in Example 1 prepared by the preparation method of the present invention, as indicated in red, characteristic new peaks may be observed. The four peaks at 600 cm⁻¹, 705 cm⁻¹, 820 cm⁻¹ and 1150 cm⁻¹ indicate that a new molecular motion mode was created in addition to the existing stretching and bending motions as a molecular association was formed.

From the spectrum results of Figures 5 and 6, the result values measured by spectroscopic analysis such as NMR or FT-IR for the nano-molecular association prepared by the preparation method of the present invention and the pharmacologically active ingredient, which is a precursor of the nano-molecular aggregate, were different from each other, and it was determined that the difference between these result values was due to the difference in physical structure of both materials. Specifically, in the case of comparing the result values measured by NMR, it could be determined that they were different when the peak position changed, and in the case of comparing the result values measured by FT-IR, it could be determined that they were different when one or more peaks appeared or disappeared, and in this case, it could be seen that the difference in the result values was caused by the difference in the physical structure of the two materials.

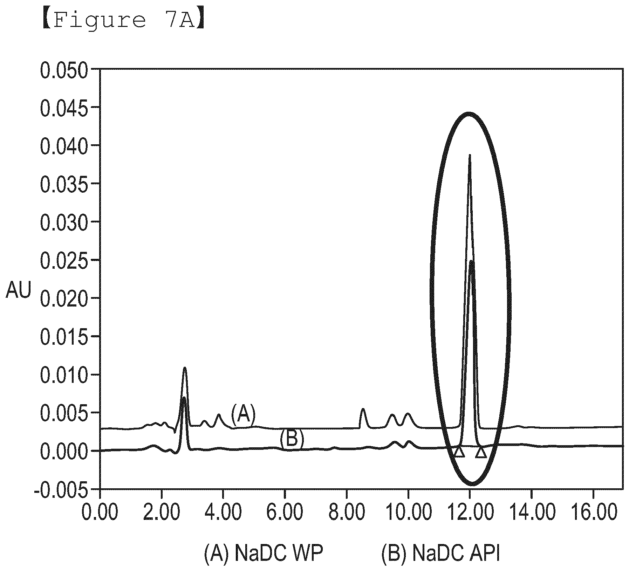

In addition, HPLC (e2695 from Waters) measurements were performed on the molecular association of NaDC in powder form prepared in Example 1 and NaDC in Comparative Example 1, and the results are shown in Figure 7A. As shown in the main peak indicated by the red ellipse in Figure 7A, the API (Comparative Example) before preparing the structure and the newly prepared molecular association by application of shear stress have the same chromatographic characteristics, and from this, it can be confirmed that they are the same material with the same peak. Therefore, as can be seen in Figure 7A, as a result of HPLC measurement, it could be seen that the molecular association of NaDC in powder form prepared in Example 1 and NaDC in Comparative Example 1 had the same result value.

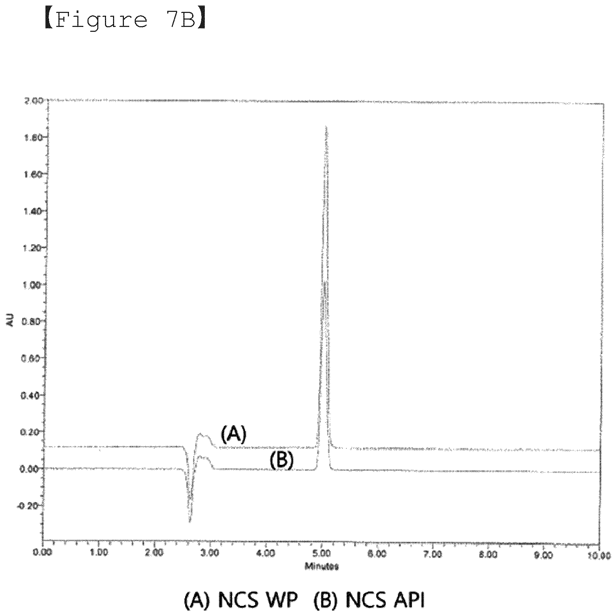

Likewise, in order to confirm that the API before preparing the molecular association and the newly prepared molecular association by application of shear stress had the same chromatographic characteristics even when the molecular association was prepared using the niclosamide prepared in Example 4 of the present application as an API, they were measured using HPLC in the same manner as in the previous Experimental Examples, and as a result, it could be seen that they had the same peak as shown in the graph of Figure 7B.

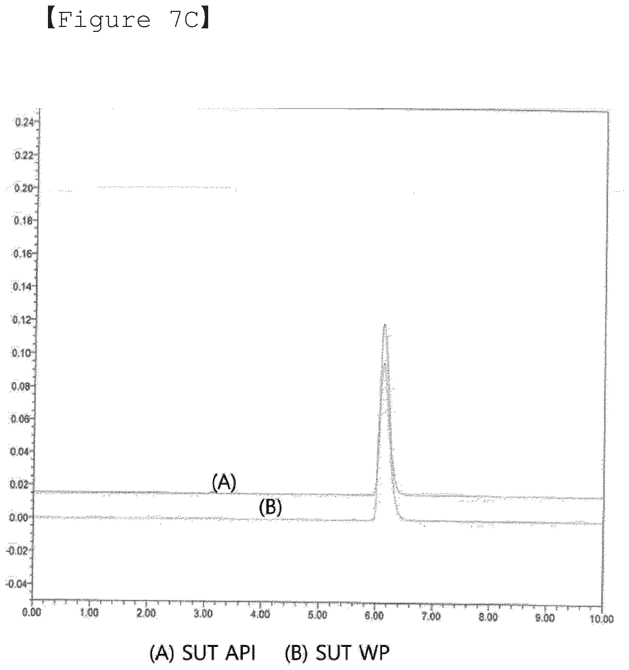

In addition, in order to confirm that the API before preparing the molecular association and the newly prepared molecular association by application of shear stress had the same chromatographic characteristics even in the case of the molecular association of sunitinib/maleate prepared in Example 2 of the present application and the salts of sunitinib and maleate prepared in Comparative Example 2, they were measured using HPLC in the same manner as in the previous Experimental Examples, and as a result, it could be seen that they had the same peak as shown in the graph of Figure 7C.

Therefore, from the results of Figure 7, the result values measured by chromatographic analysis such as HPLC for the nano-molecular association prepared by the preparation method of the present invention and the pharmacologically active ingredient, which is a precursor of the nano-molecular aggregate, were the same, and it was determined that these results were due to the same chemical structure of both materials.

In addition, looking at the two spectra shown in Figure 5, although a change was observed in terms of sharp resolution of the peak, the position of the peak was almost the same. This means that when comparing pure NaDC and the molecular association thereof newly prepared by the preparation method of the present invention, no new chemical bonds are formed or chemical bonds are removed, and they also have the same chemical structure.

From this, it can be seen that the molecular association prepared by the preparation method of the present invention has new characteristics with the same chemical structure as the pharmacologically active material, which is a precursor of the molecular aggregate, but different physical structure of the molecule.

### Experimental Example 2: Comparison of chemical/physical structures in Example 2 and Comparative Example 2

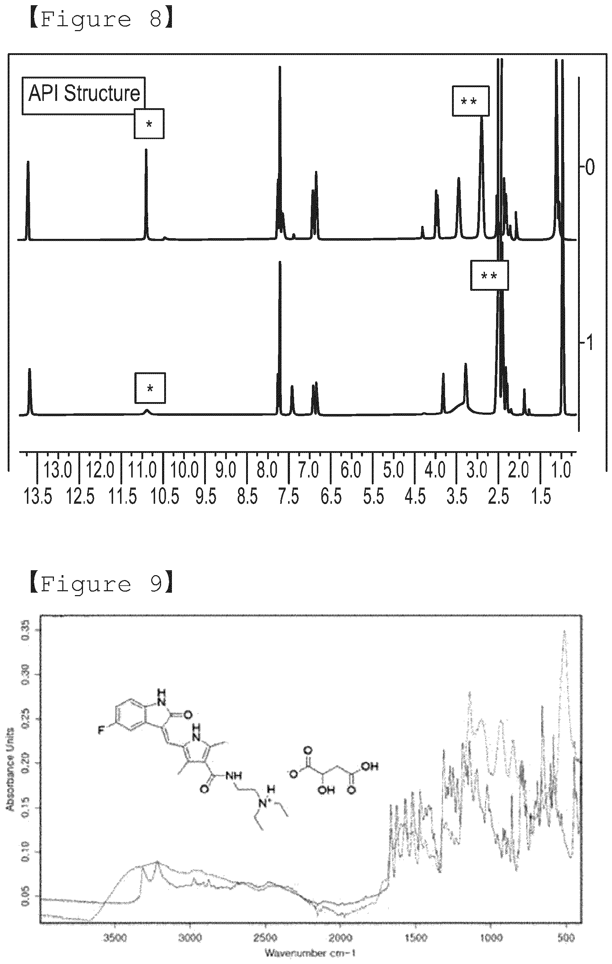

For the sunitinib/maleate molecular association prepared in Example 2 and the salts of sunitinib and maleate prepared in Comparative Example 2, the spectra were measured using NMR and FT-IR, and the spectrum results according to NMR are shown in Figure 8 and the spectrum results according to FT-IR are shown in Figure 9.

First, the H-NMR spectrum result of the salts of sunitinib/maleate and the H-NMR spectrum result of the molecular association prepared in the present invention are shown in Figure 8. In Figure 8, the top spectrum is the H-NMR spectrum of the salt-forming mixture of sunitinib and maleate, and the bottom spectrum is the H-NMR spectrum of the molecular association in which the two compounds prepared through the present invention are physically bonded. The parts where changes occur are marked with * and **, wherein as a molecular association is formed, the distance between molecules becomes very close and these close molecules cause mutual changes in the nuclear environment, and thus, these are evidence that they are close enough to observe a change in the NMR peak.

Next, the FT-IR spectrum result of the salts of sunitinib/maleate and the FT-IR spectrum result of the molecular association prepared in the present invention are shown in Figure 9. As observed in Figure 9, it can be seen that a change much more severe than that of the NMR spectrum is observed. As described above, it can be seen that the stretching and bending modes of molecules not only change the excited energy of the motion modes due to the formation of the molecular aggregate, but also change the appearance and disappearance, position shift and intensity of peaks due to these constraints. The peaks appearing at 1300-1700 cm⁻¹ represent the stretching motion of C=O appearing in the molecular structure. As can be seen from the chemical structures shown in Figure 9, there are four different carbonyl groups in the mixture of the two molecules, which are located at different positions. However, for the molecular association prepared using the mixture of these two molecules, these four sharp peaks become broad and split, and the intensity of the peaks also changes greatly. This means that several carbonyl groups are creating various environments through interactions with each other. In addition, it can be confirmed that new peaks appear near 900 cm⁻¹ and 500 cm⁻¹, and when observing the peaks for the stretching motion of OH and NH, which are known to appear in the region of 3000-3500 cm⁻¹, it can be observed that, overall, the peaks become broad, and the positions of the peaks change to such an extent that it is difficult to define the positions of the peaks. From the above, it can be seen that as the two molecules get closer to each other, the hydrogen bond is strengthened, and various peaks appear depending on the strength of the bond, and as a result, the intensity of the broad peak is increased compared to the molecular mixture.

From the spectrum results of Figures 8 and 9, the result values measured by spectroscopic analysis such as NMR or FT-IR for the nano-molecular association prepared by the preparation method of the present invention and the pharmacologically active ingredient, which is a precursor of the nano-molecular aggregate, were different from each other, and it was determined that the difference between these result values is due to the difference in physical structure of both materials. Specifically, in the case of comparing the result values measured by NMR, it could be determined that the result values measured by spectroscopic analysis were different from each other when the peak position changed, and in the case of comparing the result values measured by FT-IR, it could be determined that the result values measured by spectroscopic analysis were different from each other when one or more peaks appeared or disappeared, and in this case, it could be seen that the difference in the result values was caused by the difference in the physical structure of the two materials.

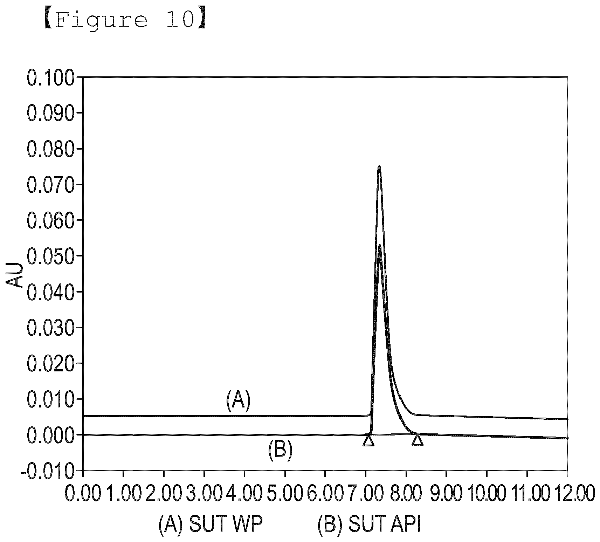

In addition, from the results of Figure 10, the result values measured by chromatographic analysis such as HPLC for the nano-molecular association prepared by the preparation method of the present invention and the pharmacologically active ingredient, which is a precursor of the nano-molecular aggregate, were the same, and it was determined that these results were due to the same chemical structure of both materials.

### Experimental Example 3: Comparison of chemical/physical structures in Example 3 and Comparative Example 3

For the powder prepared in Example 3 and the powder prepared in Comparative Example 3, the spectra were measured using (Fourier transform-infrared spectroscopy (FT-IR; Bruker Alpha 2 ATR) and compared with the spectrum result of Comparative Example 1, an original precursor not prepared by the preparation method of the present invention, which had already been measured.

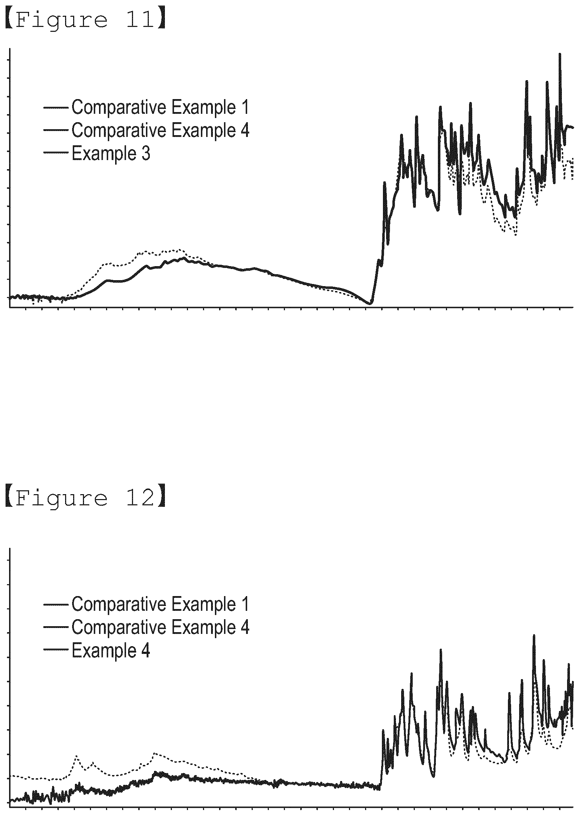

First, the comparison of the spectrum results according to FT-IR of Example 3, Comparative Example 3 and Comparative Example 1 is shown in Figure 11, and the comparison of the spectrum results according to FT-IR of Example 4, Comparative Example 4 and Comparative Example 1 is shown in Figure 12.

As shown in Figure 11, Comparative Example 3 and Comparative Example 1 showed almost similar spectrum results according to FT-IR, and thus it could be seen that when the distance between the two roll mills was adjusted to exceeding 100 µm, the molecular association according to the present invention was not prepared. Conversely, in the case of Example 3, it could be seen that when the distance between the two roll mills was adjusted to 100 um or less, a new structure with a different physical structure, i.e., the molecular association according to the present invention was prepared.

Next, as shown in Figure 12, Comparative Example 4 and Comparative Example 1 also showed almost similar spectrum results according to FT-IR, and thus it could be seen that when the distance between the two roll mills was adjusted to exceeding 100 um regardless of the type of pharmacologically active ingredient, the molecular association according to the present invention was not prepared. Conversely, in the case of Example 4, it could be seen that when the distance between the two roll mills was adjusted to 100 um or less, a new structure with a different physical structure, i.e., the molecular association according to the present invention was prepared.

Therefore, it could be seen that regardless of the type of pharmacologically active ingredient, when the distance between the two roll mills was adjusted to more than 100 µm, the molecular association according to the present invention was not produced, but when it was adjusted to 100 um or less, the molecular association according to the present invention was prepared.

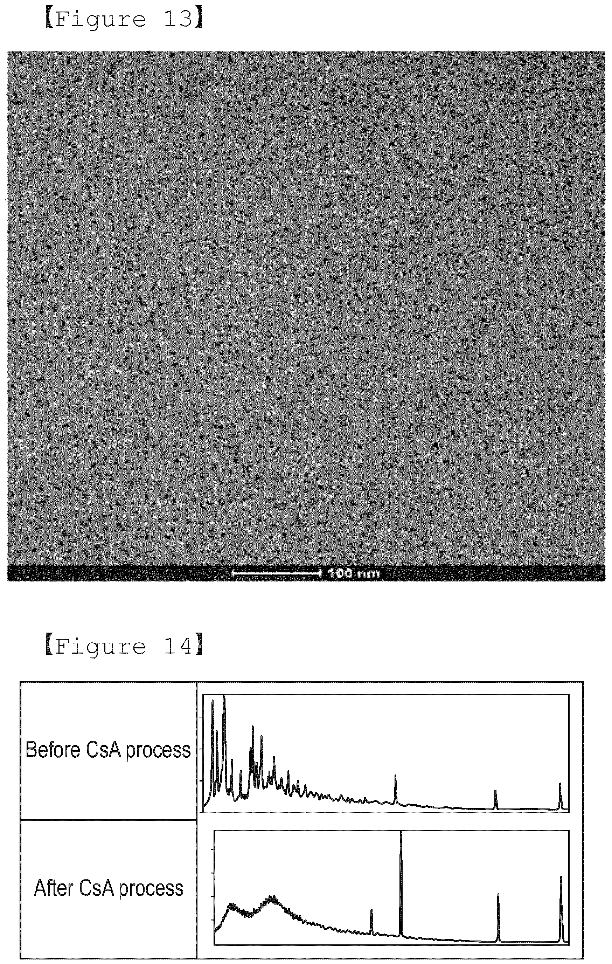

In addition, the molecular association of niclosamaide prepared in Example 4 was photographed by TEM (Tecnai G2 Spirit Twin from FEI Company) and is shown in Figure 13. The eccentricity measured using the transmission electron microscope image had an average value of about 0.85, and the distribution existed between 0.48 and 1.0. In addition, the minimum and maximum sizes were about 2.5 nm and 7.7 nm, respectively, and the average size was about 4.8 nm.

### Experimental Example 4: Stability test and confirmation of particle size for the molecular association in Example 2

The "molecular association of sunitinib/maleate" in powder form prepared in Example 2 was prepared as a 0.05% aqueous solution, and then the changes in pH, particle size, % content and concentration for 6 months were measured under the following conditions and are shown in Table 2 below.

### pH measurement method

0.5 mL of sample was taken and analyzed once. 0.5 mL of sample was put using a 1 mL pipette so that the pH meter sensor was completely covered, and the pH was measured.

### Particle size (particle size by zetasizer) measurement method

1 mL of sample was taken. The sample was placed carefully in the cuvette cell so as not to create air bubbles. A total of 10 repeated analyzes were performed on the same sample. (The default setting value is 10 repeated analysis for one measurement.) Among the processed data, part of the size distribution by volume was used, and the size and PDI values were treated with the average of 10 values.

### Zetasizer measurement conditions

**[Table 1]**

| **Temperature** | 25°C | **Duration Used** | 25 S |
|---|---|---|---|
| **Count rate** | 202.5 Kcps | **Measurement (set)** | 4.65 |
| **Cell Description** | Disposable sizing cuvette | **Attenuator** | 10 |

### Content measurement conditions Preparation of standard solution for calibration curve

20 mM ammonium acetate and acetonitrile were prepared as mobile phase. The mobile phase was flowed into the HPLC for about 30 minutes. A sunitinib sample was dissolved in the vehicle to prepare 0.005%, 0.01%, 0.03%, 0.05% and 0.1% standard solutions. Area values were measured under HPLC conditions using the standard solutions. A calibration curve was created by setting the measured area values as the y-axis and the known concentrations as the x-axis. When the calibration curve was completed, the formula for the y-axis was obtained.

### Concentration by HPLCS

1 mL of sample was taken and placed in an LC vial. The mobile phase was flowed into the HPLC for about 30 minutes. HPLC area was measured by injecting 10 µl. The concentration was calculated using the created calibration curve.

**[Table 2]**

| **Classifica tion** | **Suitabi lity criteri a** | **Sample#: SUT2111JY130** | | | | | | |
|---|---|---|---|---|---|---|---|---|
| | | **Week 0** | **Week 1** | **Week 2** | **Week 4** | **Month 2** | **Month 5** | **Month 6** |
| **pH** | - | 6.20 | 6.18 | 6.23 | 6.22 | 6.15 | 6.20 | 6.20 |
| **Particle size** | - | 2.23 nm ± 0.53 (PDI 0.52) | 2.65 nm ± 0.71 (PDI 0.29) | 2.18 nm ± 0.41 (PDI 0.70) | 2.46 nm ± 0.16 (PDI 0.71) | 3.14 nm ± 0.73 (PDI 0.20) | 2.86 nm ± 1.52 (PDI 0.19) | 3.26 nm ± 0.78 (PDI 0.14) |
| **Content (%)** | 95.0 - 115.0 % | 109.52 | 108.45 | 109.90 | 110.78 | 110.75 | 110.84 | 110.08 |

As shown in Table 2 above, the average particle size of the molecular association prepared in Example 2 was 2.2 nm, and the PDI was 1. In addition, it could be seen that the stability was maintained because the pH, content and particle size were maintained for 6 months.

### Experimental Example 5: Comparison of XRD structures in Examples and Comparative Examples

The XRD measurement results of the molecular association in Example 6 of the present invention prepared using cyclosporin A and the cyclosporin A itself in Comparative Example 6 were compared and are shown in Figure 14. As can be seen through the comparison of the graphs, except for the peaks appearing by the holder (SUS material) holding the specimen (peaks appearing identically on the right side of the graphs), it can be seen that the API (cyclosporine A) itself in Comparative Example 6 before the process exists as a crystal form (sharp peaks on the left side), whereas the molecular association in Example 6 of the present invention after the process changes to an amorphous form (amorphous peaks on the left) as the physical structure changes.

In the case of a crystalline structure rather than an amorphous structure such as the molecular association of the present application, peaks may change as the size of the internal lattice changes, but this is also just a change from the existing crystal form to another crystal form, and it does not change to an amorphous form as in the present invention.

Therefore, it can be seen from Figure 14 that the molecular association of the present invention does not change to a sorbate form, but the physical structure itself changes to an amorphous form.

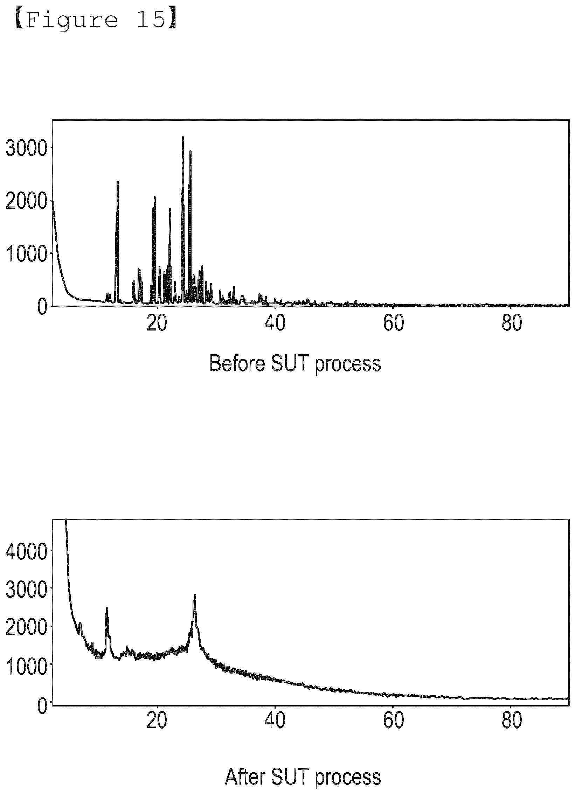

Likewise, the XRD measurement results of the molecular association in Example 2 of the present invention prepared using sunitinib/malate salt as API and Comparative Example 2 were compared and are shown in Figure 15.

Like cyclosporin A described above, it can be seen that the API (sunitinib/malate salt) itself in Comparative Example 2 before the process exists as a crystal form, whereas the molecular association in Example 2 of the present invention after the process changes to an amorphous form as the physical structure changes. That is, it can be seen that the molecular association of the present invention does not change to a sorbate form, but the physical structure itself changes to an amorphous form.

### Experimental Example 6: Comparison of DSC measurement experiments of Examples and Comparative Examples

In order to clearly prove that the molecular association of the present application was not crystallized (sorbated), the applicant of the present application conducted the following DSC measurement experiment.

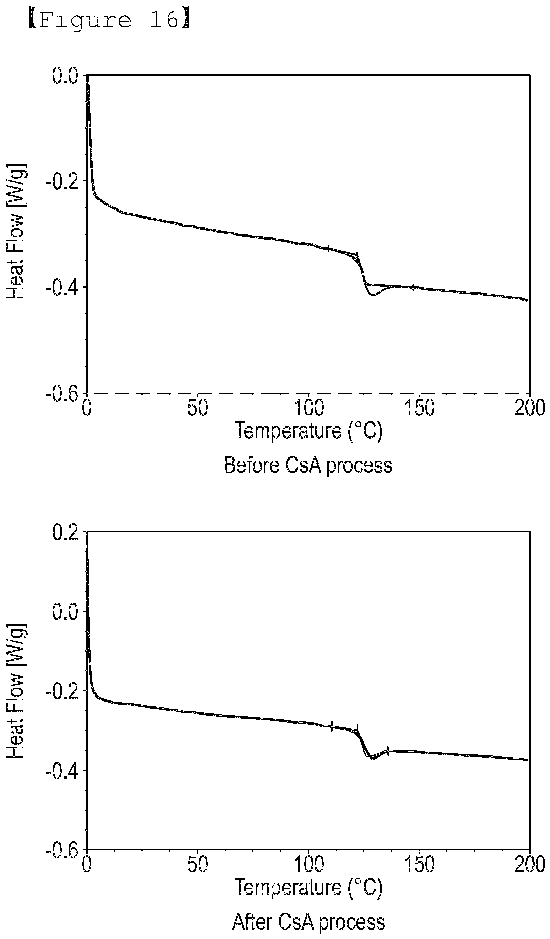

Specifically, the DSC measurement results of the molecular association in Example 6 of the present invention prepared using cyclosporin A as API and the cyclosporin A itself in Comparative Example 6 were compared and are shown in Figure 16. If the molecular association that has undergone the process of the present invention is sorbate, a peak in which the solvent (the solvent used in the present invention is water or ethanol) blows away near the boiling point (100°C for water, about 60°C for ethanol) should appear on the DSC graph, but such a peak does not appear at all in the DSC measurement result data shown in Figure 16. Therefore, it can be clearly seen that the difference between the measured values by spectroscopy of the molecular association of the present application and the pharmacologically active ingredient, which is a precursor of the nano-molecular aggregate, is due to a change in physical structure, not a change caused by sorbation.

### Experimental Example 7: Confirmation of particle pictures of Examples

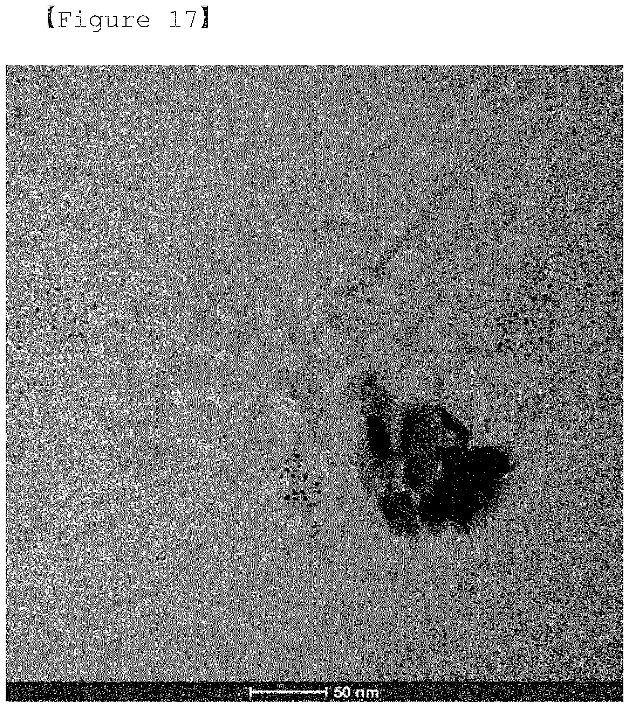

In order to confirm that the molecular association of the present application is a molecular association structure of API, the applicant of the present application took TEM pictures of the molecular association in Example 6 prepared using cyclosporin A as API and the molecular association in Example 2 prepared using sunitinib/malate salt. First, a TEM picture of the molecular association in Example 6 prepared using cyclosporin A as API was taken and is shown in Figure 17. Cyclosporin A itself cannot be confirmed by TEM because it is not well soluble in water, but as can be seen in the TEM picture of Figure 17, it can be confirmed that the molecular structure according to the present invention is a structure in which several APIs are physically bonded.

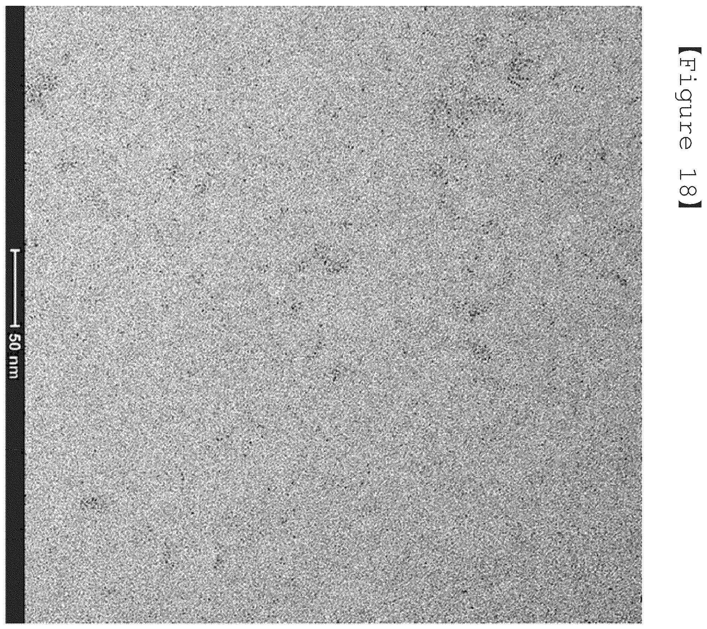

Likewise, a TEM picture of the molecular association in Example 2 prepared using sunitinib/malate salt was taken and is shown in Figure 18.

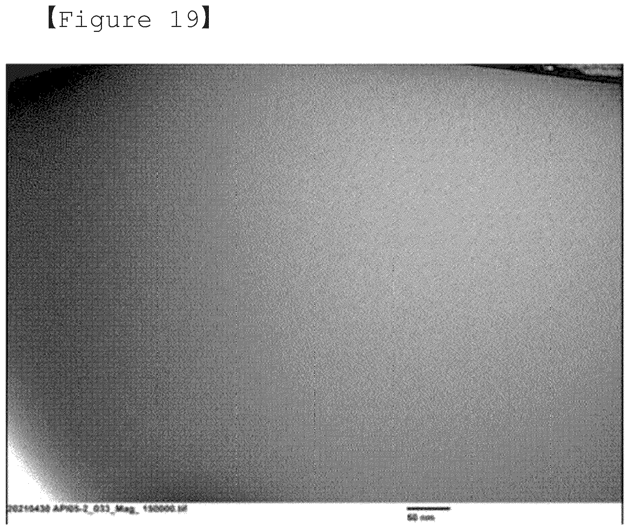

As can be seen in the TEM picture of Figure 18, it can be confirmed that the molecular association according to the present invention is a structure in which several APIs are physically bonded. On the other hand, as shown in the TEM picture of Figure 19, looking at the result of taking a TEM picture of Comparative Example 2, which is the sunitinib/malate salt itself dissolved in water, the structure in which the APIs are physically bonded could not be confirmed at all.

Therefore, it can be clearly seen that the structure according to the present invention is a molecular association structure in which a plurality of APIs are physically bonded.

### Experimental Example 8: Comparison of cell permeation performance of Example 5 and Comparative Example 5

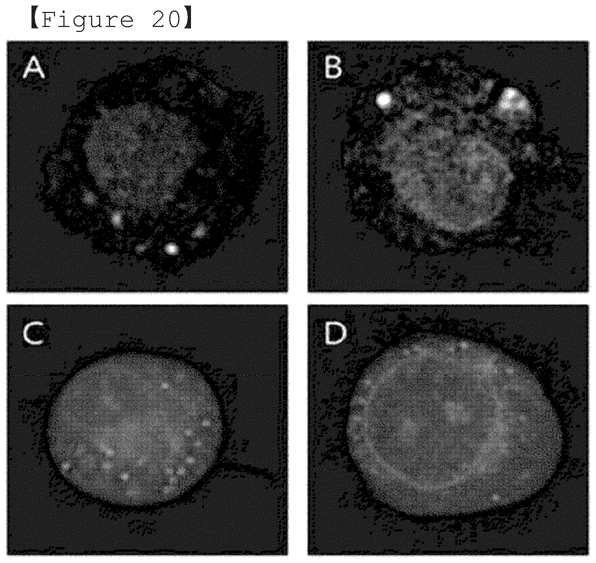

The cell permeation performance of the DCF-DA molecular association in powder form prepared in Example 5 and the DCF-DA itself in Comparative Example 5 was compared. Suspension cells (MV-4-11 human macrophages) were grown in dishes to fill up to 50%. DCF-DA in Comparative Example 5 was dissolved in ethanol, and the DCF-DA molecular association in Example 5 was dissolved in distilled water, and the cells were treated with each of them at the same concentration of 0.005% for 30 minutes, and then the cells were washed with phosphate buffer solution (PBS) to remove extracellular DCF-DA and to stop the entry by diffusion. The cells were stabilized by culture for an additional 30 minutes, and then treated with 0.03% hydrogen peroxide (H₂O₂), and intracellular fluorescent DCFs were photographed by 3D hologram and fluorescence using a 3D microscope (HT-2H from Tomocube, Inc.), and are shown in Figure 20. As a result of the experiment, it could be confirmed that the DCF-DA molecular aggregate-treated group in Example 5 as shown in C and D of Figure 20 showed much stronger fluorescence compared to the DCF-DA-treated group in Comparative Example 5 as shown in A and B of Figure 20. Therefore, it could be confirmed that the permeability of the molecular association prepared according to the present invention into the phospholipid membrane was much higher.

### Experimental Example 9: Comparison of corneal permeation performance of Example 2 and Comparative Example 2

A corneal permeation experiment of the sunitinib-malate molecular association in powder form prepared in Example 2 and the sunitinib-malate itself in Comparative Example 2 was performed.

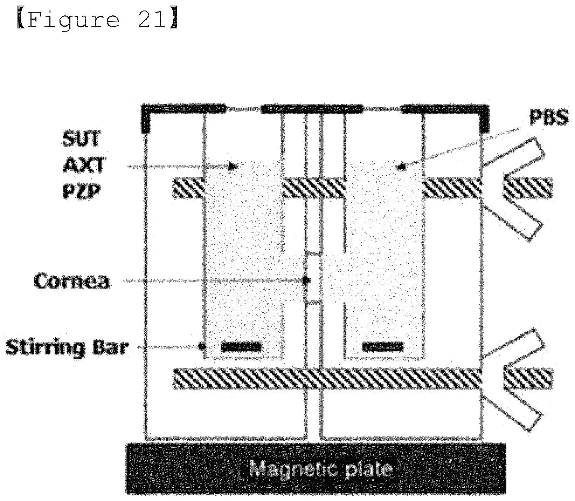

The corneal permeation experiment was conducted with reference to the contents of "Lee et. al, "Modulating the Transport Characteristics of Bruch's Membrane With Steroidal Glycosides and its Relevance to Age-Related Macular Degeneration (AMD). Invest. Ophthalmol. Vis. Sci. 2015, 56, 8403., 2015," which is an already published paper. As a cell for the permeation experiment, a Ussing chamber was self-manufactured using as shown in Figure 21, and the Ussing chamber is largely composed of three parts: a donor chamber for administering drugs, a receiver chamber, and a membrane through which the drug permeates. Basically, the drug is put into the donor chamber of the Ussing chamber, the corneal part of the pig eye is collected in a circular shape of 8 mm in size, and other tissues attached to the cornea were removed, and then fixed in place of the membrane, and the solution permeating the membrane was received in the receiving chamber, and at this time, it was placed in a constant temperature chamber in which the temperature at which diffusion occurs was adjusted to 37°C, which is close to human body temperature, and stored. After a certain amount of time passed, the diffusion properties of each sample could be identified by measuring the concentration of the solution in each donor chamber and receiving chamber. The concentration after 18 hours for each sample is shown in Table 1 below. For the concentration, UV/VIS spectrum (Evolution201 UV/Vis; Thermo) was used, and the concentration was measured using a peak height at 420 nm.

**[Table 3]**

| | **Initial drug load** (µg) | **Drug in corneal tissue** (µg, Mean±SD) | **Drug in receiver** (µg, Mean±SD) | **Transport percentage*** (%, Mean±SD) |
|---|---|---|---|---|
| **Comparative Example 2** | 540 | 65.7 ± 7.5 | 8.8 ± 5.8 | 1.6 ± 1.1 |
| **Example 2** | 490 | 70.2 ± 10.7 | 27.8 ± 2.9 | 5.7 ± 0.6*** |

As shown in Table 3 above, when the permeated amount is expressed as a percentage, it can be seen that the permeability of the molecular association of the pharmacologically active material is approximately 4 times greater than that of the pharmacologically active material. Therefore, as observed in the cell permeation experiment of DCF-DA in Example 4 above, it could be seen that the permeation of the molecular association according to the present invention into cells was much better than that of the pharmacologically active material itself.

## Claims

1. An amorphous nano-molecular association in which an organic material or an inorganic material is physically bonded,
wherein when formed as a composition comprising water in the nano-molecular aggregate, the nano-molecular association in the composition has an aggregated structure, and
wherein the average particle diameter of the nano-molecular association is 50 nm or less.

2. The amorphous nano-molecular association according to claim 1, **characterized in that**:
the organic material or inorganic material itself and the nano-molecular association itself have the same result values as measured by high-performance liquid chromatography (HPLC), and
the organic material or inorganic material itself and the nano-molecular association itself have different result values as measured by NMR or FT-IR method.

3. The amorphous nano-molecular association according to claim 1, **characterized in that** the result values measured by HPLC of the organic material or inorganic material itself and the nano-molecular association itself are determined to be the same when they have a retention time within 10%.

4. The amorphous nano-molecular association according to claim 1, **characterized in that** in comparison of the result values measured by NMR of the organic material or inorganic material itself and the nano-molecular association itself, they are determined to be different when peak positions change.

5. The amorphous nano-molecular association according to claim 4, **characterized in that** in comparison of the result values measured by NMR of the organic material or inorganic material itself and the nano-molecular association itself, they are determined to be different when peak shifts are 0.005 ppm or more based on 1H NMR.

6. The amorphous nano-molecular association according to claim 1, **characterized in that** in comparison of the result values measured by FT-IR of the organic material or inorganic material itself and the nano-molecular association itself, they are determined to be different when one or more peaks appear or disappear.

7. The amorphous nano-molecular association according to claim 1, **characterized in that** in comparison of the result values measured by FT-IR of the organic material or inorganic material itself and the nano-molecular association itself, they are determined to be different when one or more peak positions differ by 5 cm⁻¹ or more.

8. The amorphous nano-molecular association according to claim 1, **characterized in that** the organic material or inorganic material is any one or more pharmacologically active ingredients selected from the group consisting of cyclosporine A, paclitaxel, docetaxel, decursin, meloxicam, itraconazole, celecoxib, capecitabine, travo, prost, isoflavone, diclofenac sodium, sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, deoxycholic acid, ursodeoxycholic acid, sodium or potassium salt of deoxycholic acid, sodium or potassium salt of ursodeoxycholic acid, prednisolone, eucalyptus oil, lavender oil, lemon oil, sandalwood oil, rosemary oil, chamomile oil, cinnamon oil, orange oil, alpha-bisabolol, vitamin A (retinol), vitamin E, tocopheryl acetate and vitamin D, vitamin F, and derivatives thereof.

9. The amorphous nano-molecular association according to claim 1, **characterized in that** the nano-molecular association has an intermolecular distance of 10 Å or less.

10. The amorphous nano-molecular association according to claim 1, **characterized in that** the nano-molecular association is composed of the organic material or inorganic material.

11. An amorphous nano-molecular association in which a salt of organic material or inorganic material is physically bonded,
wherein when formed as a composition comprising water in the nano-molecular aggregate, the nano-molecular association in the composition has an aggregated structure, and
wherein the average particle diameter of the nano-molecular association is 50 nm or less.

12. The amorphous nano-molecular association according to claim 11, **characterized in that**:
the salt of organic material or inorganic material itself and the nano-molecular association itself have the same result values as measured by high-performance liquid chromatography (HPLC), and
the salt of organic material or inorganic material itself and the nano-molecular association itself have different result values as measured by NMR or FT-IR method.

13. The amorphous nano-molecular association according to claim 11, **characterized in that** the result values measured by HPLC of the salt of organic material or inorganic material itself and the nano-molecular association itself are determined to be the same when they have a retention time within 10%.

14. The amorphous nano-molecular association according to claim 11, **characterized in that** in comparison of the result values measured by NMR of the salt of organic material or inorganic material itself and the nano-molecular association itself, they are determined to be different when peak positions change.

15. The amorphous nano-molecular association according to claim 14, **characterized in that** in comparison of the result values measured by NMR of the salt of organic material or inorganic material itself and the nano-molecular association itself, they are determined to be different when peak shifts are 0.005 ppm or more based on 1H NMR.

16. The amorphous nano-molecular association according to claim 11, **characterized in that** in comparison of the result values measured by FT-IR of the salt of organic material or inorganic material itself and the nano-molecular association itself, they are determined to be different when one or more peaks appear or disappear.

17. The amorphous nano-molecular association according to claim 11, **characterized in that** in comparison of the result values measured by FT-IR of the salt of organic material or inorganic material itself and the nano-molecular association itself, they are determined to be different when one or more peak positions differ by 5 cm⁻¹ or more.

18. The amorphous nano-molecular association according to claim 11, **characterized in that** the salt of organic material or inorganic material is pharmacologically active ingredients that are salts of any one or more organic materials or inorganic materials selected from the group consisting of cyclosporine A, paclitaxel, docetaxel, decursin, meloxicam, itraconazole, celecoxib, capecitabine, travo, prost, isoflavone, diclofenac sodium, sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, deoxycholic acid, ursodeoxycholic acid, sodium or potassium salt of deoxycholic acid, sodium or potassium salt of ursodeoxycholic acid, prednisolone, eucalyptus oil, lavender oil, lemon oil, sandalwood oil, rosemary oil, chamomile oil, cinnamon oil, orange oil, alpha-bisabolol, vitamin A (retinol), vitamin E, tocopheryl acetate and vitamin D, vitamin F, and derivatives thereof.

19. The amorphous nano-molecular association according to claim 11, **characterized in that** the nano-molecular association has an intermolecular distance of 10 Å or less.

20. The amorphous nano-molecular association according to claim 11, **characterized in that** the nano-molecular association is composed of the salt of organic material or inorganic material.

21. A method for preparing the nano-molecular association according to claim 1 or 10, comprising steps of:
putting a composition comprising an organic material, an inorganic material or a salt thereof, and water into an apparatus capable of applying a shear stress; and then,
applying a shear stress to the composition to prepare a nano-molecular association in which an organic material, an inorganic material or a salt thereof is physically bonded.

22. The method for preparing the nano-molecular association according to claim 21, **characterized in that**:
the apparatus is provided with a plurality of rolls to apply a shear stress to a solution containing the organic material, inorganic material or salt thereof;
the solution containing the organic material, inorganic material or salt thereof is put between two rolls facing each other in the apparatus; and then,
a shear stress is applied to the solution containing the organic material, inorganic material or salt thereof.

23. The method for preparing the nano-molecular association according to claim 22, **characterized in that** the distance between the two rolls facing each other is 0.5 to 1000 um.

24. The method for preparing the nano-molecular association according to claim 23, **characterized in that** the two rolls facing each other rotate at different speeds.

25. The method for preparing the nano-molecular association according to claim 24, **characterized in that** the rotational speed of any one of the two rolls facing each other is 50 to 250 rpm, and the rotational speed of the other is 200 to 500 rpm.

26. The method for preparing the nano-molecular association according to claim 21, **characterized in that** the organic material or inorganic material is any one or more pharmacologically active ingredients selected from the group consisting of cyclosporine A, paclitaxel, docetaxel, decursin, meloxicam, itraconazole, celecoxib, capecitabine, travo, prost, isoflavone, diclofenac sodium, sunitinib, pazopanib, axitinib, regorafenib, trametinib, ginsenoside Rg1, tacrolimus, alendronate, latanoprost, bimatoprost, atorvastatin calcium, rosuvastatin calcium, entecavir, amphotericin B, omega-3, deoxycholic acid, ursodeoxycholic acid, sodium or potassium salt of deoxycholic acid, sodium or potassium salt of ursodeoxycholic acid, prednisolone, eucalyptus oil, lavender oil, lemon oil, sandalwood oil, rosemary oil, chamomile oil, cinnamon oil, orange oil, alpha-bisabolol, vitamin A (retinol), vitamin E, tocopheryl acetate and vitamin D, vitamin F, and derivatives thereof.
